# NEW EUROPEAN PATENT SPECIFICATION

(11) **EP 1 786 893 B2**
(45) Date of publication and mention of the opposition decision: **02.11.2016**
(45) Mention of the grant of the patent: 19.12.2012
(21) Application number: 05786818.4
(22) Date of filing: 17.08.2005
(51) Int. Cl.: C11D 17/00, A61K 8/00

(54) **LOW PH STRUCTURED SURFACTANT COMPOSITIONS**
STRUKTURVISKOSE TENSIDZUSAMMENSETZUNGEN MIT NIEDRIGEM PH-WERT
COMPOSITIONS TENSIOACTIVES STRUCTUREES A PH FAIBLE

(30) Priority: 17.08.2004 US 602156 P
(43) Date of publication of application: 23.05.2007
(73) Proprietor: Solvay USA Inc., Cranbury, NJ 08512 (US)
(72) Inventor: FRANTZ, Seren, Bensalem, PA 19020 (US); WARBURTON, Stewart, Alexander, West Windsor, NJ 08550 (US)
(74) Representative: Gillard, Richard Edward
(86) International application number: PCT/US2005/029240
(87) International publication number: WO 2006/023548

(56) References cited:
- EP-A- 0 160 342
- EP-A- 0 271 189
- WO-A-01/70926
- WO-A-99/32069
- DE-A1- 3 837 985
- DE-A1- 10 255 991
- DE-A1- 10 258 960
- DE-A1- 10 259 014
- GB-A- 2 179 053
- US-B1- 6 177 396

## Description

### Field of the invention

This invention relates to personal care compositions comprising surfactant compositions, more particularly to personal care compositions comprising low pH structured liquid surfactant compositions.

### Background of the Invention

Structured surfactant compositions are pumpable fluid compositions that exhibit shear-thinning viscosity and have the capacity physically to suspend water insoluble or partially water soluble ingredients. Typically, the surfactant phase is present as packed spherulites, i.e., lamellar droplets, formed from an aqueous solution, see eg. WO 01/709 26 and WO 99/32069.

Structured surfactant compositions are useful in personal care applications, such as shampoos, body wash, hand soap, lotions, creams, conditioners, shaving products, facial washes, neutralizing shampoos, and skin treatments, in home care applications, such as liquid detergents, laundry detergents, hard surface cleansers, dish wash liquids, toilet bowl cleaners, and in other applications, such as oil field and agrochemical applications.

Structured surfactant compositions for the personal care market typically exhibit a pH in the range about 5 to 7. The surfactants used in known structured surfactant compositions are, in many cases, unsuitable for use in low pH systems, because the typical components of such compositions become unstable and ineffective at a pH of less than about 5.

In some applications, it would be desirable to suspend water insoluble or partially water soluble ingredients in a low pH aqueous composition. For example, U.S. Patent No. 6,416,768 discloses the use of water-in-oil emulsions to incorporate active ingredients in low pH compositions. However, water-in-oil emulsions are capable of suspending only lipophilic agents and typically have limited stability over time.

What is needed is a structured surfactant composition that provides typical structured surfactant properties, that is, shear-thinning viscosity and a capacity to suspend water insoluble or partially water soluble components, at low pH.

### Summary of the Invention

In a first aspect, the present invention is directed to claim 1.

In a second aspect, the present invention is directed to claim 15.

### Detailed Description of Invention and Preferred Embodiments

As used herein in reference to viscosity, the terminology "shear-thinning" means that such viscosity decreases with an increase in shear rate. Shear-thinning may be characterized as a "non-Newtonian" behavior, in that it differs from that of a classical Newtonian fluid, for example, water, in which viscosity is not dependent on shear rate.

As used herein in reference to a component of an aqueous composition, the terminology "water insoluble or partially water soluble components" means that the component is present in the aqueous composition at a concentration above the solubility limit of the component so that, in the case of a water insoluble component, the component remains substantially non-dissolved in the aqueous composition and, in the case of a partially water soluble component, at least a portion of such component remains undissolved in the aqueous composition.

As used herein, characterization of an aqueous composition as "capable of suspending", or as being "able to suspend" water insoluble or partially water insoluble components means that the composition substantially resists flotation of such components in the composition or sinking of such components in such composition so that such components appear to be neutrally buoyant in such composition and remain at least substantially suspended in such composition under the anticipated processing, storage, and use conditions for such low pH aqueous composition.

As used herein in reference to a component of a low pH aqueous composition, the terminology "chemically stable" means that the component remains at least substantially non-degraded under the anticipated processing, storage, and use conditions for such low pH aqueous composition.

As used herein, the terminology "lamellar phase" means a phase that comprises a plurality of bilayers of surfactant arranged in parallel and separated by liquid medium. A lamellar phase is detectable by, for example, small angle x-ray measurement or by evidence of birefringence under a cross-polarized microscope. Lamellar phases include both spherulitic phases and the typical form of the liquid crystal G-phase, as well as mixtures thereof. "G-phases", which are sometimes referred to in the literature a L_{α} phases, are typically pumpable, non-Newtonian, anisotropic products that are cloudy looking and exhibit a characteristic "smeary" appearance on flowing. Lamellar phases, can exist in several different forms, including domains of parallel sheets which constitute the bulk of the typical G-phases described above and spherulites formed from a number of concentric spheroidal shells, each of which is a bilayer of surfactant. In this specification the term "G-phase" will be reserved for compositions which are at least partly of the former type. The spherulites are typically between 0.1 and 50 microns in diameter and so differ fundamentally from micelles. Unlike micellar solutions, spherulitic compositions are typically anisotropic and non-Newtonian. When close packed, spherulites have good solid suspending properties and allow incorporation of water insoluble or partially water soluble solids, liquids and/or gases as a separate, discontinuous phase suspended in a continuous matrix of the surfactant composition.

The low pH structured liquid surfactant composition exhibits a pH of from 3 to 4.

The structured surfactant composition comprises, based on 100 pbw of the structured surfactant composition, from about 3 to about 40 pbw, more typically from about 5 to about 30 ppw, and still more typically from about 8 to about 20 pbw, of the anionic surfactants.

Compounds suitable as the anionic surfactant component of the present invention are anionic phosphate surfactants, such as monoalkyl phosphate surfactants and dialkyl phosphate surfactants. In one embodiment, the alkyl substituent of such monoalkyl phosphate surfactants and dialkyl phosphate surfactants is a (C₈-C₂₄)alkyl group, which may be branched or linear.

Anionic surfactants that are suitable as the anionic surfactant component of the composition of the present invention include, for example, lauryl phosphate, cetyl phosphate, tridecyl phosphate, behenyl phosphate, laureth-2 phosphate, ceteth-3 phosphate, trideceth-4 phosphate, trideceth-6 phosphate, beheneth-4 phosphate, dilauryl, phosphate, dicetyl phosphate, ditridecyl phosphate, dibehenyl phosphate, dilaureth-2 phosphate, dipareth-3 phosphate, di-ceteth-4 phosphate, ditrideceth-4 phosphate, ditrideceth-6 phosphate, or mixtures thereof wherein the anionic surfactant may be included in the formulation in the acid (non-neutralized form) or as the neutralized salt.

When neutralized, the cation of any anionic surfactant is typically sodium but may alternatively be potassium, lithium, calcium, magnesium, ammonium, (C₁-C₆)alkyl ammonium, or (C₁-C₆)alkanol ammonium, such as isopropylammonium, monoethanolammonium, diethanolammonium, and triethanolammonium. Ammonium and ethanolammonium salts are generally more soluble that the sodium salts. Mixtures of the above cations may be used.

In one embodiment, the anionic surfactant comprises non-neutralized acids of one or more phosphate esters, such as, for example, Dermalcare MAP L-210 (laureth-2 phosphate) and Rhodifac RS-410 (trideceth-4 phosphate). Such surfactants, when dispersed in water, will readily form a lamellar phase at a low pH, without requiring a structurant. The propensity to form a lamellar phase makes such surfactants particularly useful in formulating low pH structured liquid formulations.

In one embodiment, the structured surfactant composition optionally comprises at least an effective amount of one or more structuring agents. Suitable structuring agents which can have chemical stability at low pH include cationic surfactants, such as amine salts, quaternary ammonium compounds, and amine oxides, nonionic surfactants, such as fatty alcohols, ethoxylated alcohols, and fatty acids, and electrolytes. An effective amount of such structuring agent is one that can aid in the formation of a shear-thinning phase capable of suspending water insoluble or partially water soluble components.

Suitable cationic surfactants are known compounds. Any cationic surfactant that is acceptable for use in the intended end use application and is chemically stable at the required formulation pH is suitable as a structurant component of the composition of the present invention, including, for example, cationic surfactants according to formula (1) below: 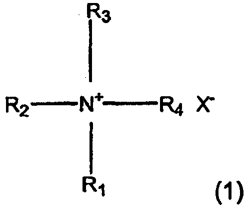 wherein:
R₁, R₂, R₃ and R₄, are independently hydrogen, an organic group, provided that at least one of R₁, R₂, R₃ and R₄ is not hydrogen, and
X⁻ is an anion.

If one to three of the R groups are hydrogen, the compound may be referred to as an amine salt. Some examples of cationic amines include polyethoxylated (2) oleyl/stearyl amine, ethoxylated tallow amine, cocoalkylamine, oleylamine, and tallow alkyl amine.

For quaternary ammonium compounds (generally referred to as quats) R₁. R₂, R₃, and R₄ may be the same or different organic group, but may not be hydrogen. In one embodiment, R₁, R₂, R₃, and R₄ are each (C₈-C₂₄₎ branched or linear which may comprise additional functionality such as, for example, fatty acids or derivatives thereof, including esters of fatty acids and fatty acids with alkoxylated groups, alkyl amido groups, aromatic rings, heterocyclic rings, phosphate groups, epoxy groups, and hydroxyl groups. The nitrogen atom may also be part of a heterocyclic or aromatic ring system, e.g., cetethyl morpholinium ethosulfate or steapyrium chloride.

Suitable anions include, for example, chloride, bromide, methosulfate, ethosulfate, lactate, saccharinate, acetate or phosphate.

Examples of quaternary ammonium compounds of the monoalkyl amine derivative type include: cetyl trimethyl ammonium bromide (also known as CETAB or cetrimonium bromide), cetyl trimethyl ammonium chloride (also known as cetrimonium chloride), myristyl trimethyl ammonium bromide (also known as myrtrimonium bromide or Quaternium-13), stearyl dimethyl benzyl ammonium chloride (also known as stearalkonium chloride), oleyl dimethyl benzyl ammonium chloride, (also known as olealkonium chloride), lauryl/myristryl trimethyl ammonium methosulfate (also known as cocotrimonium methosulfate), cetyl-dimethyl-(2)hydroxyethyl ammonium dihydrogen phosphate (also known as hydroxyethyl cetyldimonium phosphate), bassuamidopropylkonium chloride, cocotrimonium chloride, distearyldimonium chloride, wheat germamidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate.

Quaternary ammonium compound of the dialkyl amine derivative type distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride and mixtures thereof.

Quaternary ammonium compounds of the imidazoline derivative type include, for example, isostearyl benzylimidonium chloride, cocoyl benzyl hydroxyethyl imidazolinium chloride, cocoyl hydroxyethylimidazolinium PG-chloride phosphate, Quaternium 32, and stearyl hydroxyethylimidonium chloride, and mixtures thereof.

Suitable cationic surfactants include, for example, amine salts such as polyethoxylated (2) oleyl/stearyl amine, ethoxylated tallow amine, cocoalkylamine, oleylamine, and tallow alkyl amine or quaternary ammonium compounds such as cetyl trimethyl ammonium bromide (also known as CETAB or cetrimonium bromide), cetyl trimethyl ammonium chloride (also known as cetrimonium chloride), myristyl trimethyl ammonium bromide (also known as myrtrimonium bromide or Quaternium-13), stearyl dimethyl benzyl ammonium chloride (also known as stearalkonium chloride), oleyl dimethyl benzyl ammonium chloride, (also known as olealkonium chloride), lauryl/myristryl trimethyl ammonium methosulfate (also known as cocotrimonium methosulfate), cetyl-dimethyl-(2)hydroxyethyl ammonium dihydrogen phosphate (also known as hydroxyethyl cetyldimonium phosphate), bassuamidopropylkonium chloride, cocotrimonium chloride, distearyldimonium chloride, wheat germ-amidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate, distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride, isostearyl benzylimidonium chloride, cocoyl benzyl hydroxyethyl imidazolinium chloride, cocoyl hydroxyethylimidazolinium PG-chloride phosphate, Quaternium 32, and stearyl hydroxyethylimidonium chloride, and mixtures thereof.

Additionally, amine oxides may be used as structuring agents due to their cationic nature at low pH. Specific examples of suitable alkyl amine oxides may include, for example, lauramine oxide, cocamine oxide, cocamidopropylamine oxide, and lauramidopropylamine oxide.

Nonionic surfactants are known. Any nonionic surfactant that is acceptable for use in the intended end use application and is chemically stable at the required formulation pH is suitable as a structurant component of the composition of the present invention.

Suitable fatty alcohols include, for example, (C₁₀-C₂₂) saturated or unsaturated branched or straight chain alcohols, such as for example, decyl alcohol, lauryl alcohol, tridecyl alcohol, myristyl alcohol, cetyl alcohol, stearyl alcohol, oleyl alcohol, linoleyl alcohol and linolenyl alcohol.

Suitable ethoxylated alcohols include alkoxylated, typically ethoxylated, derivatives of (C₁₀-C₂₂) saturated or unsaturated branched or straight chain alcohols, which may include, on average, from 1 to 22 alkoxyl units per molecule of ethoxylated alcohol, for example, laureth-1 laureth-2 laureth-4, laureth-5, laureth-7, laureth-9, trideceth-1, trideceth-2, trideceth-3, (C₁₁-C₁₅)pareth-3, (C₁₂-C₁₃)pareth-5, and (C₁₄-C₁₅)pareth-9.

Suitable fatty acids include (C₁₀-C₂₂) saturated or unsaturated acids, such as, for example, lauric acid, oleic acid, stearic acid, isostearic acid, myristic acid, cetearic acid, isostearic acid, linoleic acid, linolenic acid, ricinoleic acid, elaidic acid, arichidonic acid, myristoleic acid, palmitoleic acid, or the neutralized versions thereof.

Electrolytes suitable as a structurant component of the composition of the present invention include salts of multivalent anions, such as potassium pyrophosphate, potassium tripolyphosphate, and sodium or potassium citrate, salts of multivalent cations, including alkaline earth metal salts such as calcium chloride and calcium bromide, as well as zinc halides, barium chloride and calcium nitrate, salts of monovalent cations with monovalent anions, including alkali metal or ammonium halides, such as potassium chloride, sodium chloride, potassium iodide, sodium bromide, and ammonium bromide, alkali metal or ammonium nitrates, and polyelectrolytes, such as uncapped polyacrylates, polymaleates, or polycarboxylates, lignin sulphonates or naphthalene sulphonate formaldehyde copolymers.

Typically, the greater the amount of anionic surfactant present in relation to its solubility, the lesser the amount of structurant required in order to form a structure capable of supporting solid materials and/or to cause flocculation of the structured surfactant. The structurant is incorporated in an amount sufficient to, in combination with the one or more anionic surfactants, promote the formation of structured surfactant composition that exhibits shear-thinning viscosity, and is capable of suspending water insoluble or partially water soluble components and may be added separately or may be included in one of the other raw materials added to the formulation.

In one embodiment, the structured surfactant composition of the present invention comprises, based on 100 pbw of the structured surfactant composition, up to about 40 pbw, more typically from about 0.5 to about 25 pbw and still more typically from about 1 to about 10 pbw of one or more structurants.

In another embodiment, the structured surfactant composition of the present invention does not require a structurant to form the lamellar phase.

In one embodiment, the structured surfactant composition of the present invention comprises less than an effective amount of structuring agent. In another embodiment, the structured surfactant composition of the present invention does not comprise a structurant.

The composition of the present invention may optionally further comprise, in addition to the anionic surfactant and any cationic surfactant, nonionic surfactant and/or electrolyte used as a structuring agent, one or more cationic surfactants, one or more non-ionic surfactants, one or more electrolytes, one or more amphoteric surfactants, one or more zwitterionic surfactants, or a mixture thereof. Such optional cationic surfactant, nonionic surfactant and/or electrolyte may each independently be present in an amount in excess of the minimum amount effective to act as a structurant.

The cationic surfactants, nonionic surfactant, and electrolytes discussed above are also suitable as the respective optional additional cationic surfactant, nonionic surfactant and electrolyte.

Suitable Zwitterionic surfactants are known compounds. Any Zwitterionic surfactant that is acceptable for use in the intended end use application and is chemically stable at the required formulation pH is suitable as the optional Zwitterionic surfactant component of the composition of the present invention, including, for example, those which can be broadly described as derivatives of aliphatic quaternary ammonium, phosphonium, and sulfonium compounds in which the aliphatic radicals can be straight chain or branched and wherein one of the aliphatic substituents contains from about 8 to about 24 carbon atoms and one contains an anionic water-solubilizing group such as carboxyl, sulfonate, sulfate, phosphate or phosphonate. Specific examples of suitable Zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxyethyl)sulfopropyl betaine and alkylamidopropylhydroxy sultaines.

Suitable amphoteric surfactants are known compounds. Any Amphoteric surfactant that is acceptable for use in the intended end use application and is chemically stable at the required formulation pH is suitable as the optional amphoteric surfactant component of the composition of the present invention, including, for example, those which can be broadly described as compounds in which the aliphatic radicals can be straight chain or branched and wherein one of the aliphatic substituents contains from about 8 to about 24 carbon atoms and one contains an anionic water-solubilizing group such as carboxyl, sulfonate, phosphate or phosphonate. Specific examples of suitable amphoteric surfactants include alkyl propionates, such as cocoampho propionates, lauroapho propionates, tridecylampho propionate, oleylampho propionates, caprylampho propionates, behenylampho propionates, dipropionates such as cocoampho dipropionates, lauroapho dipropionates, tridecylampho dipropionate, oleylampho dipropionates, caprylampho dipropionates, behenylampho dipropionates, and amphoteric sulfonates such as cocoampho hydroxypropyl sulfonates, lauroampho hydroxypropyl sulfonates, tridecylampho hydroxypropyl sulfonates, oleylampho hydroxypropyl sulfonates, caprylampho hydroxypropyl sulfonates, behenylampho hydroxypropyl sulfonates.

In one embodiment, the composition of the present invention comprises, based on 100 pbw of the composition and inclusive of any surfactant used as a structuring agent, a total amount of from about 0.1 to about 20 pbw, more typically from about 0.5 to about 15 pbw, and still more typically from about 1 to about 10 pbw, of one or more cationic surfactants, nonionic surfactants, amphoteric surfactants, and/or zwitterionic surfactants.

In one embodiment, the structured surfactant composition is made by combining and mixing a surfactant and water and optionally, adjusting the pH and then adding any optional structurant. Mixing may be applied as required to form a homogeneous solution.

In one embodiment, the structured surfactant is subjected to a high shear mixing in known mixing equipment, such as, for example, a high shear mixer or a homogenizer.

Shear-thinning viscosity is measured by known viscometric methods, such as for example, using a rotational viscometer, such as a Brookfield viscometer. In one embodiment, the composition of the present invention exhibits shear-thinning behavior when subjected to viscosity measurement using a Brookfield rotational viscometer, equipped with an appropriate spindle, at a rotation speed of from about 0.1 revolutions per minute ("rpm") to about 60 rpm.

The composition of the present invention is capable of suspending water-insoluble particles or partially water soluble components, such as vegetable oils, mineral oils, silicone oils, solid particles, abrasives, and similar articles. The composition provides a means to include otherwise difficult to incorporate components in surfactant mixtures resulting in cosmetic preparations with multi-functional benefits including, in some cases, cleansing, moisturizing, improved skin feel, exfoliation/abrasion, novel appearance, or a combination of these benefits.

The ability of a composition to suspend water insoluble or partially water insoluble components is typically evaluated by mixing the composition with sufficient vigor to entrap air bubbles in the composition and then visually observing whether the air bubbles remain entrapped in the composition for a defined period of time, such as for example, 12 to 24 hours, under defined environmental conditions, such as for example, room temperature. In one embodiment, the composition of the present invention is capable of suspending air bubbles for at least 1 week, and more typically for at least 3 months. A composition that is capable of suspending air bubbles under the for at least 12 hours at room temperature is deemed to be generally capable of suspending water insoluble or partially water soluble components in the composition under generally anticipated processing, storage, and use conditions for such composition. For components other than air, the result of the air suspension test should be confirmed by conducting an analogous suspension test using the component of interest. For unusually rigorous processing, storage and/or use conditions, more rigorous testing may be appropriate.

In one embodiment, the ability to suspend water insoluble or partially water insoluble components is evaluated under more rigorous conditions, that is, the mixed samples are visually evaluated after subjecting the samples to one or more freeze/thaw cycles, wherein each freeze/thaw cycle consists of 12 hours at -10°C and 12 hours at 25°C. In one embodiment, composition of the present invention remains capable of suspending air bubbles after one freeze/thaw cycle, more typically after 3 freeze/thaw cycles.

In one embodiment, the composition of the present invention further comprises one or more water insoluble or partially water soluble components. Such components may be in the form of a solid, a liquid, or a gas and may comprise one or more materials selected from water insoluble or partially water soluble chemically stable benefit agents, such as, for example, in the case of a personal care application, emollients, conditioners, moisturizers, vitamins, vitamin derivatives, anti-UV agents, anti-bacterial agents, anti-fungal agents, tanning accelerators, anti-aging agents, anti-wrinkle agents, antiperspirants, deodorants, essential oils, fragrances, air, or abrasives, and water insoluble or partially water soluble chemically stable appearance modifying additives such as, for example, colored or reflective particles or beads.

In another embodiment, the personal care composition of the present invention comprises the structured surfactant component as defined in claim 1 that forms a first phase (which may itself comprise a plurality of phases, including aqueous phases, laminar surfactant phases, and spherulitic phases, as discussed above) and the composition further comprises one or more additional phases that are at least substantially distinct from such first phase. As used herein in reference to the phases of a multiphase embodiments of the present invention, the terminology "substantially distinct" means that the phases each exhibit substantially homogeneous prop- erties within a given phase and that the phases differ with respect to at least one characteristic or property, such as for example, visual characteristics, such as color, clarity, pearlescence, or physical/chemical properties, such as viscosity, lubricity, and/or benefit agent content

In one embodiment, the structured surfactant component forms a first phase that exhibits shear-thinning viscosity and is capable of suspending water insoluble or partially water soluble components.

In one embodiment, the structured surfactant component forms a first phase that exhibits shear-thinning viscosity and is capable of suspending water insoluble or partially water soluble components and the composition comprises at least one additional phase that is at least substantially distinct from such first phase and that does not exhibit shear-thinning viscosity and/or is not capable of suspending water insoluble or partially water soluble components.

In one embodiment, the structured surfactant component forms a first phase that exhibits shear-thinning viscosity and is capable of suspending water insoluble or partially water soluble components and the composition comprises at least one additional phase, such as an additional structured surfactant component, that is at least substantially distinct from such first phase and that exhibits shear-thinning viscosity and is capable of suspending water insoluble or partially water soluble components.

In one embodiment, the structured surfactant component forms a first phase and the composition comprises at least one additional phase that is at least substantially distinct from such first phase, wherein each of the phases is a continuous phase and the phases are disposed adjacent to each other.

In one embodiment, the structured surfactant component forms a first phase and the composition comprises at least one additional phase that is at least substantially distinct from such first phase, wherein one of such phases is a continuous phase, the other phase is a discontinuous phase, and the discontinuous phase is disposed adjacent to or is dispersed within the continuous phase.

In one embodiment, the structured surfactant component forms a first phase and the composition comprises at least one additional phase that that is at least substantially visually distinct from such first phase such as for example, wherein at least one additional phase is an opaque water insoluble component that is suspended in the first phase.

In one embodiment, the composition of the present invention comprises two distinct phases, wherein each of the phases is a continuous phase and the phases are disposed adjacent to each other.

In one embodiment, the structured surfactant composition comprises two distinct phases, wherein one phase is a continuous phase the other phase is a discontinuous phase and the discontinuous phase is disposed adjacent to the continuous phase or is dispersed within the continuous phase.

In one embodiment, the structured surfactant composition comprises two distinct phases, wherein each phase is a continuous phase and the two phases are disposed in a mutually interpenetrating network.

In one embodiment, the structured surfactant composition comprises two or more visually distinct phases, such as for example, two or more visually distinct phases which exhibit an appearance of alternating visually distinct stripes.

Examples of personal care applications include shampoos, body washes, hand soap, lotions, creams, conditioners, shaving products, facial washes, neutralizing shampoos, and skin treatments

The personal care composition of the present invention comprises a structured surfactant composition and an aqueous carrier.

In one embodiment, the personal care composition of the present invention further comprises one or more chemically stable benefit agents, such as emollients, moisturizers, conditioners, skin conditioners, hair conditioners, vitamins or their derivatives, antioxidants, free-radical scavengers, abrasives, dyes, hair coloring agents, bleaching agents, hair bleaching agents, anti-UV agents, UV absorbers, antimicrobial agents, antibacterial agents, antifungal agents, melanin regulators, tanning accelerators, depigmenting agents, skin-coloring agents, liporegulators, weight-reduction agents, anti-acne agents, antiseborrhoeic agents, anti-ageing agents, anti-wrinkle agents, keratolytic agents, anti-inflammatory agents, refreshing agents, cicatrizing agents, vascular-protection agents, antiperspirants, deodorants, immunomodulators, nourishing agents, agents for combating hair loss, reducing agents for permanent-waving, essential oils and fragrances.

In one embodiment, the personal care composition of the present invention further comprises one or more chemically stable benefit agents selected from acids ,such as, ascorbic acid, salicylic acid, alpha-hydroxy acids, betahydroxy acids, alpha-keto acids optionally in lactone form, kojic acid, caffeic acid, physic acid, quinic acid, benzene-1,4bis(3-methylidenecamphorsulfonic acid, glycolic acid, lactic acid, mandelic add, malic acid, tartaric acid, citric acid, hydroxybutyric acid, gluconic acid, ascorbic acid, salicylic acid, gentisic acid, homogentisic acid, and pyruvic acid, zinc pyrithion, Vitamin B, Vitamin E Acetate, silicone fluids, organosilicon materials, such as, silicone gums, polyorganosiloxane fluids, and crosslinked polyorganosiloxane resins, hydrocarbon conditioning agents, such as petrolatum, mineral oils, and gelled mineral oils, thickening and/or benefit polymers, such as succinoglycan (Rheozan from Rhodia), methyl cellulose products such as carboxymethyl cellulose gum (Aqualon CMC-7HOF from Aqualon), modified starches such as sodium hydroxypropyl starch phosphate (Pure-Gel 980 and Pure-Gel 998 from Grain Processing Corporation), potato starch modified (Structure-Solanace from National Starch), acrylates copolymers such as Acrytates/Aminoacrylates/C10-30 Alkyl PEG-20 Itaconate Copolymer (Structure-Plus from National Starch), cationic polymers (Rheovis CSP, Rheovis CDE, Rheovis CDP from Ciba), Polyacrylimidomethylpropane Sulfonate / Polyquaternium-4 (Plexagel ASC from ISP), hydrohobically modified nonionic polyols (Acusol 880, Acusol 882 from Rohm & Haas).

In one embodiment, the personal care composition of the present invention further comprises up to about 40 pbw, more typically from about 0.1 to about 40 pbw, even more typically from about 0.3 to about 20 pbw, and still more typically from about 0.5 to 10 pbw, of one or more chemically stable benefit agents.

The personal care composition of the present invention may, optionally, further comprise other ingredients, such as, for example, preservatives, such as benzyl alcohol, methyl paraben, propyl paraben and imidazolidinyl urea, viscosity adjusting agents, electrolytes, such as sodium chloride, and sodium sulfate, pH adjusting agents such as citric acid, succinic acid, phosphoric acid, sodium hydroxide, potassium hydroxide, and sodium carbonate, perfumes, colorants, and sequestering agents, such as disodium ethylene diamine tetra-acetate.

In general, personal care composition of the present invention may optionally comprise, based on 100 pbw of the personal care composition and independently for each such ingredient, up to about 10 pbw, preferably from 0.5 pbw to about 5.0 pbw, of such other ingredients, depending on the desired properties of the personal care composition.

### EXAMPLES 1-3

The compositions of Examples 1-3 were made by mixing the relative amounts of the ingredients listed in TABLE I, and allowing them to sit overnight at ambient lab temperature. Typically, the monoalkyl phosphate a ("MAP") component was first dissolved in water, followed by the pH adjustment, where applicable. All amounts are indicated by the pbw per 100 pbw of the composition. The mixed compositions were visually observed to contain entrapped air bubbles.

**TABLE I**

| **Ingredient** | **Ex. 1** | **Ex. 2** | **Ex. 3** |
|---|---|---|---|
| Laureth-1 Phosphate (Dermalcare MAP L-210, Rhodia) | 24.9 | 24.7 | 24.5 |
| 50% NaOH | 0.5 | 1.0 | 2 |
| water | 74.6 | 74.3 | 73.5 |

The compositions of Examples 1-3 were each evaluated as being capable of suspending air, based on the visual observation that the samples contained entrapped air bubbles after sitting overnight. The compositions of Examples 1-3 were each subjected to one freeze/thaw cycle (12 hours at -10°C and 12 hours at 25°C). and evaluated as having retained the capacity to suspend air following the freeze/thaw cycle, based on the visual observation that samples contained entrapped air bubbles after having been subjected to the freeze thaw cycle.

### EXAMPLES 4-7

The compositions of Examples 4-7 were made by mixing the relative amounts of the ingredients listed in TABLE II, and allowing them to sit overnight at ambient lab temperature. Typically, the MAP ester was dissolved in water, followed by the pH adjustment, where applicable. All amounts are indicated by the pbw per 100 pbw of the composition.

**TABLE II**

| **Ingredient** | **Ex. 4** | **Ex. 5** | **Ex. 6** | **Ex. 7** |
|---|---|---|---|---|
| Laureth-1 Phosphate (Dermalcare MAP L-210, Rhodia) | 16.7 | 16.4 | 24.9 | 8.3 |
| Isostearic Acid | 1.3 | 1.3 | | |
| Cetrimonium Bromide (Rhodaquat M242B/99, Rhodia) | 0.9 | 0.9 | 0.5 | 0.17 |
| Laureth-2 (Genapol 26-L-2) | 7.1 | 8.7 | | |
| Succinoglycan (Rheozan, Rhodia) | | | 1.33 | 1.33 |
| 50% NaOH | 1.8 | 1.8 | | |
| water | 72.2 | 70.9 | 74.6 | 90.2 |

The compositions of Examples 4-7 were evaluated for the ability to suspend air using the procedure described above in reference to the compositions of Examples 1-3 and were each found to be capable of suspending air and to retain the capacity to suspend air following the freeze/thaw cycle.

### EXAMPLES 8-10

The compositions of Examples 8-10 were made by mixing the relative amounts of the ingredients listed in TABLE III, and allowing them to sit overnight at ambient lab temperature. Typically, the MAP ester was dissolved in water, followed by the pH adjustment, other surfactant additions, and salt addition, where applicable. All amounts are indicated by the pbw per 100 pbw of the composition.

**TABLE III**

| **Ingredient** | **Ex. 8** | **Ex. 9** | **Ex. 10** |
|---|---|---|---|
| Trideceth-4 Phosphate (Rhodifac RS-410, Rhodia) | 12.2 | 12.6 | 12.4 |
| Trideceth-7 Carboxylic Acid (Nikkol ECT-7, Nikko) | | | 3.8 |
| Lauryl Betaine (Mirataine BB/FLA, Rhodia) | 4.9 | 5.0 | 5.0 |
| Laureth-2 (Genapol 26-L-2) | 4.6 | | |
| Isostearic Acid | | 1.9 | |
| Sodium Chloride | 3.6 | 3.7 | 2.8 |
| 50% NaOH | 1.3 | 1.3 | 1.3 |
| water | 73.4 | 75.5 | 74.8 |

The compositions of Examples 8-10 were evaluated for the ability to suspend air using the procedure described above in reference to the compositions of Examples 1-3 and were each found to be capable of suspending air and to retain the capacity to suspend air following the freeze/thaw cycle.

### EXAMPLES 11-12

The composition of Examples 11-12 were made by mixing the relative amounts of the ingredients listed in TABLE IV, and allowing them to sit overnight at ambient lab temperature. Typically, the MAP ester was dissolved in water, followed by the pH adjustment, where applicable. All amounts are indicated by the pbw per 100 pbw of the composition.

**TABLE IV**

| **Ingredient** | **Ex. 11** | **Ex. 12** |
|---|---|---|
| Trideceth-4 Phosphate (Rhodifac RS-410, Rhodia) | 22.9 | 16.0 |
| Dodecyl Benzene Sulfonic Acid (Rhodacal SSA/A, Rhodia) | 1.6 | 1.1 |
| 50% NaOH | 0.5 | 0.3 |
| water | 75.0 | 82.5 |

The compositions of Examples 11-12 were evaluated for the ability to suspend air using the procedure described above in reference to the compositions of Examples 1-3 and were each found to be capable of suspending air and to retain the capacity to suspend air following the freeze/thaw cycle.

### EXAMPLES 13-15

The composition of Examples 13-15 were made by mixing the relative amounts of the ingredients listed in TABLE V, and allowing them to sit overnight at ambient lab temperature. Typically, the MAP ester was dissolved in water, followed by the pH adjustment, nonionic surfactant and sodium chloride, where applicable. All amounts are indicated by the pbw per 100 pbw of the composition.

**TABLE V**

| **Ingredient** | **Ex. 13** | **Ex. 14** | **Ex. 15** |
|---|---|---|---|
| Laureth-2 Phosphate (Dermalcare MAP L-210, Rhodia) | 9.1 | 11.3 | 9.1 |
| Trideceth-4 Phosphate (Rhodifac RS-410, Rhodia) | 4.7 | | 4.7 |
| Laureth-2 (Genapol 26-L-2) | 6.0 | 13.0 | 6.0 |
| Sodium Chloride | 0.6 | | 0.4 |
| 10% NaOH | 7.3 | 9.1 | 7.3 |
| water | 72.4 | 66.6 | 72.6 |

The compositions of Examples 13-15 were evaluated for the ability to suspend air using the procedure described above in reference to the compositions of Examples 1-3 and were each found to be capable of suspending air and to retain the capacity to suspend air following the freeze/thaw cycle.

## Claims

1. A personal care composition comprising an aqueous, low pH structured surfactant composition, comprising, based on 100 parts by weight of the aqueous, low pH structured surfactant composition, from about 3 parts by weight to about 40 parts by weight anionic phosphate ester surfactants wherein the aqueous, low pH structured surfactant composition exhibits a pH of 3 to 4, exhibits shear-thinning viscosity, and is capable of suspending water insoluble or partially water soluble components; and
wherein the total amount of anionic phosphate ester surfactants and anionic carboxylate surfactants is from about 3 parts by weight to about 40 parts by weight.

2. The personal care composition of claim 1, wherein the anionic surfactant is selected from monoalkyl phosphate surfactants, dialkyl phosphate surfactants, and mixtures thereof.

3. The personal care composition of claim 1, wherein at least a portion of the aqueous, low pH structured surfactant composition is in the lamellar form.

4. The personal care composition of claim 1, further comprising a structuring agent selected from cationic surfactants, nonionic surfactants, electrolytes and mixtures thereof.

5. The personal care composition of claim 1, further comprising a structuring agent selected from amine salts, quaternary ammonium compounds, amine oxides, fatty alcohols, ethoxylated alcohols, fatty acids, electrolytes, and mixtures thereof

6. The personal care composition of claim 1, wherein the aqueous, low pH structured surfactant composition comprises, based on 100 parts by weight of the aqueous, low pH structured surfactant composition, from about 0.5 to about 25 parts by weight structuring agent.

7. The personal care composition of claim 1, further comprising a surfactant selected from nonionic surfactants, amphoteric surfactants, zwitterionic surfactants, cationic surfactants, and mixtures thereof.

8. The personal care composition of claim 1, wherein the personal care composition is selected from shampoos, body washes, hand soaps, lotions, creams, conditioners, shaving products, facial washes, and skin treatments

9. The personal care composition of claim 1, further comprising one or more water insoluble or partially water soluble components suspended in the composition.

10. The personal care composition of claim 1, further comprising one or more of benefit agents.

11. The personal care composition of claim 10, wherein the one or more benefit agents are selected from emollients, moisturizers, conditioners, skin conditioners, hair conditioners, vitamins or their derivatives, antioxidants, free-radical scavengers, abrasives, dyes, hair coloring agents, bleaching agents, hair bleaching agents, anti-UV agents, UV absorbers, antimicrobial agents, antibacterial agents, antifungal agents, melanin regulators, tanning accelerators, depigmenting agents, skin-coloring agents, liporegulators, weight-reduction agents, anti-acne agents, antiseborrhoeic agents, anti-ageing agents, anti-wrinkle agents, keratolytic agents, anti-inflammatory agents, refreshing agents, cicatrizing agents, vascular-protection agents, antiperspirants, deodorants, immunomodulators, nourishing agents, agents for combating hair loss, reducing agents for permanent-waving, essential oils and fragrances, and mixtures thereof

12. The personal care composition of claim 1, wherein the personal care composition comprises, based on 100 parts by weight of the personal care composition, from about 0.1 to about 40 parts by weight benefit agent.

13. The composition of claim 1, wherein the personal care composition comprises two or more visually distinct phases.

14. The composition of claim 13, wherein the two or more phases are visible as stripes.

15. A personal care composition comprising an aqueous, low pH composition, comprising at least two at least substantially distinct phases, wherein at least one of such phases is a structured surfactant composition as defined in claim 1,
and wherein the low pH structured surfactant composition exhibits a pH of 3 to 4.

## Patentansprüche

1. Körperpflegezusammensetzung, umfassend eine wässrige, strukturierte Tensidzusammensetzung mit niedrigem pH, die, bezogen auf 100 Gewichtsteile der wässrigen, strukturierten Tensidzusammensetzung mit niedrigem pH, von etwa 3 Gewichtsteile bis etwa 40 Gewichtsteile anionische Phosphatester-Tenside,
wobei die wässrige, strukturierte Tensidzusammensetzung mit einem niedrigen pH einen pH von 3 bis 4 zeigt, scherentzähende Viskosität zeigt und wasserunlösliche oder teilweise wasserlösliche Komponenten suspendieren kann; und
wobei die Gesamtmenge anionischer Phosphatester-Tenside und anionischer Carboxylat-Tenside von etwa 3 Gewichtsteile bis etwa 40 Gewichtsteile beträgt.

2. Körperpflegezusammensetzung nach Anspruch 1, wobei das anionische Tensid aus Monoalkylphosphat-Tensiden, Dialkylphosphat-Tensiden und Mischungen davon ausgewählt ist.

3. Körperpflegezusammensetzung nach Anspruch 1, wobei mindestens ein Teil der wässrigen, strukturierten Tensidzusammensetzung mit niedrigem pH in lamellarer Form vorliegt.

4. Körperpflegezusammensetzung nach Anspruch 1, die ferner ein strukturgebendes Mittel umfasst, das aus kationischen Tensiden, nichtionischen Tensiden, Elektrolyten und Mischungen davon ausgewählt ist.

5. Körperpflegezusammensetzung nach Anspruch 1, die ferner ein strukturgebendes Mittel umfasst, das aus Aminsalzen, quartären Ammoniumverbindungen, Aminoxiden, Fettalkoholen, ethoxylierten Alkoholen, Fettsäuren, Elektrolyten und Mischungen davon ausgewählt ist.

6. Körperpflegezusammensetzung nach Anspruch 1, wobei die wässrige, strukturierte Tensidzusammensetzung mit niedrigem pH, bezogen auf 100 Gewichtsteile der wässrigen, strukturierten Tensidzusammensetzung mit niedrigem pH, von etwa 0,5 bis etwa 25 Gewichtsteile an strukturbildendem Mittel umfasst.

7. Körperpflegezusammensetzung nach Anspruch 1, die ferner ein Tensid umfasst, das aus nichtionischen Tensiden, amphoteren Tensiden, zwitterionischen Tensiden, kationischen Tensiden und Mischungen davon ausgewählt ist.

8. Körperpflegezusammensetzung nach Anspruch 1, wobei die Körperpflegezusammensetzung aus Folgenden ausgewählt ist: Shampoos, Körperreinigungsmitteln, Handseifen, Lotionen, Cremes, Konditioniermitteln, Rasierprodukten, Gesichtsreinigungsmitteln und Hautbehandlungen.

9. Körperpflegezusammensetzung nach Anspruch 1, die ferner eine oder mehrere wasserunlösliche oder teilweise wasserlösliche Komponenten umfasst, die in der Zusammensetzung suspendiert ist/sind.

10. Körperpflegezusammensetzung nach Anspruch 1, die ferner ein oder mehrere begünstigende Mittel umfasst.

11. Körperpflegezusammensetzung nach Anspruch 10, wobei das eine oder die mehreren begünstigenden Mittel aus Folgenden ausgewählt sind:
Emollienzien, Feuchtigkeitsmitteln, Konditioniermitteln, Haut-Konditioniermitteln, Haar-Konditioniermitteln, Vitaminen oder ihren Derivaten, Antioxidantien, Radikalfängern, abrasiven Stoffen, Farbstoffen, Haarfärbemitteln, Bleichmitteln, Haarbleichmitteln, UV-Schutzmitteln, UV-Absorbern, antimikrobiellen Mitteln,
antibakteriellen Mitteln, antimykotischen Mitteln, Melaninregulatoren, Bräunungsbeschleunigern, Depigmentierungsmitteln,-Hautfärbemitteln,
fettregulierenden Mitteln, Schlankheitsmitteln, Mitteln gegen Akne, Antiseborrhöika, Mitteln gegen Hautalterung, Mitteln gegen Falten, Keratolytika,
entzündungshemmenden Mitteln, kühlend wirkenden Mitteln, Wundheilungsmitteln, Gefäßschutzmitteln, Antitranspirants, Deodorants, Immunmodulatoren, Nährstoffen, Mitteln gegen Haarausfall, Reduktionsmitteln für Dauerwelle, etherischen Ölen und Duftstoffen und Mischungen davon.

12. Körperpflegezusammensetzung nach Anspruch 1, wobei die Körperpflegezusammensetzung, bezogen auf 100 Gewichtsteile der Körperpflegezusammensetzung, von etwa 0,1 bis etwa 40 Gewichtsteile an begünstigendem Mittel umfasst.

13. Zusammensetzung nach Anspruch 1, wobei die Körperpflegezusammensetzung zwei oder mehr visuell unterscheidbare Phasen umfasst.

14. Zusammensetzung nach Anspruch 13, wobei die zwei oder mehr Phasen als Streifen sichtbar sind.

15. Körperpflegezusammensetzung, umfassend eine wässrige Zusammensetzung mit niedrigem pH, die mindestens zwei mindestens im Wesentlichen unterscheidbare Phasen umfasst, wobei mindestens eine von derartigen Phasen eine strukturierte Tensidzusammensetzung wie in Anspruch 1 definiert ist,
und wobei die strukturierte Tensidzusammensetzung mit niedrigem pH einen pH von 3 bis 4 zeigt.

## Revendications

1. Composition de soins d'hygiène personnelle comprenant une composition tensioactive structurée à faible pH aqueuse, comprenant, sur la base de 100 parties en poids de la composition tensioactive structurée à faible pH aqueuse, d'environ 3 parties en poids à environ 40 parties en poids de tensioactifs anioniques sélectionnés parmi des tensioactifs d'ester de phosphate anioniques
dans laquelle la composition tensioactive structurée à faible pH aqueuse exhibe un pH de 3 à 4, exhibe une viscosité structurelle, et est capable de mettre en suspension des composants insolubles dans l'eau ou partiellement solubles dans l'eau ; et
dans laquelle la quantité totale de tensioactifs d'ester de phosphate anioniques et de tensioactifs carboxylate anioniques va d'environ 3 parties en poids à environ 40 parties en poids.

2. Composition de soins d'hygiène personnelle selon la revendication 1, dans laquelle le tensioactif anionique est sélectionné parmi des tensioactifs de monoalkyle phosphate, des tensioactifs de dialkyle phosphate, et des mélanges de ceux-ci.

3. Composition de soins d'hygiène personnelle selon la revendication 1, dans laquelle au moins une portion de la composition tensioactive structurée à faible pH aqueuse est sous la forme lamellaire.

4. Composition de soins d'hygiène personnelle selon la revendication 1, comprenant en outre un agent structurant sélectionné parmi des tensioactifs cationiques, des tensioactifs non ioniques, des électrolytes et des mélanges de ceux-ci.

5. Composition de soins d'hygiène personnelle selon la revendication 1, comprenant en outre un agent structurant sélectionné parmi des sels d'amine, des composés d'ammonium quaternaire, des oxydes d'amine, des alcools gras, des alcools éthoxylés, des acides gras, des électrolytes, et des mélanges de ceux-ci.

6. Composition de soins d'hygiène personnelle selon la revendication 1, dans laquelle la composition tensioactive structurée à faible pH aqueuse comprend, sur la base de 100 parties en poids de la composition tensioactive structurée à faible pH aqueuse, d'environ 0,5 à environ 25 parties en poids d'agent structurant.

7. Composition de soins d'hygiène personnelle selon la revendication 1, comprenant en outre un tensioactif sélectionné parmi des tensioactifs non ioniques, des tensioactifs amphotères, des tensioactifs zwittérioniques, des tensioactifs cationiques, et des mélanges de ceux-ci.

8. Composition de soins d'hygiène personnelle selon la revendication 1, dans laquelle la composition de soins d'hygiène personnelle est sélectionnée parmi des shampoings, des nettoyants corporels, des savons pour les mains, des lotions, des crèmes, des après-shampoings, des produits de rasage, des nettoyants pour le visage, et des traitements pour la peau.

9. Composition de soins d'hygiène personnelle selon la revendication 1, comprenant en outre un ou plusieurs composants insolubles dans l'eau ou partiellement solubles dans l'eau mis en suspension dans la composition.

10. Composition de soins d'hygiène personnelle selon la revendication 1, comprenant en outre un ou plusieurs agents bénéfiques.

11. Composition de soins d'hygiène personnelle selon la revendication 10, dans laquelle l'un ou les plusieurs agents bénéfiques sont sélectionnés parmi des émollients, des hydratants, des conditionneurs, des conditionneurs pour la peau, des conditionneurs pour les cheveux, des vitamines, ou leurs dérivés, des antioxydants, des phagocytes de radicaux libres, des abrasifs, des colorants, des agents de coloration pour les cheveux, des agents éclaircissants, des agents éclaircissants pour les cheveux, des agents anti-UV, des absorbeurs UV, des agents antimicrobiens, des agents antibactériens, des agents antifongiques, des régulateurs de la mélanine, des accélérateurs de bronzage, des agents de dépigmentation, des agents de coloration de la peau, des liporégulateurs, des agents d'amaigrissement, des agents anti-acné, des agents anti-séborrhéiques, des agents antivieillissement, des agents antirides, des agents kératolytiques, des agents anti-inflammatoires, des agents rafraîchissants, des agents cicatrisants, des agents de protection vasculaire, des antiperspirants, des déodorants, des immunomodulateurs, des agents nourrissants, des agents pour combattre la chute des cheveux, des agents réducteurs pour les permanentes, des huiles essentielles et parfums, et des mélanges de ceux-ci.

12. Composition de soins d'hygiène personnelle selon la revendication 1, dans laquelle la composition de soins d'hygiène personnelle comprend, sur la base de 100 parties en poids de composition de soins d'hygiène personnelle, d'environ 0,1 à environ 40 parties en poids d'agent bénéfique.

13. Composition de soins d'hygiène personnelle selon la revendication 1, dans laquelle la composition de soins d'hygiène personnelle comprend deux phases visuellement distinctes ou plus.

14. Composition de soins d'hygiène personnelle selon la revendication 13, dans laquelle les deux phases ou plus sont visibles comme des bandes.

15. Composition de soins d'hygiène personnelle comprenant une composition à faible pH aqueuse, comprenant au moins deux phases au moins essentiellement distinctes, dans laquelle au moins une de ces phases est une composition tensioactive structurée telle que définie dans la revendication 1,
et dans laquelle la composition tensioactive structurée à faible pH exhibe un pH de 3 à 4.
